# EUROPEAN PATENT APPLICATION

(11) **EP 2 940 450 A1**
(43) Date of publication of application: **04.11.2015**
(21) Application number: 14001536.3
(22) Date of filing: 30.04.2014
(51) Int. Cl.: G01N 1/40, G01N 33/68, H01J 49/04

(54) **Interface for online bioaffinity-mass spectrometry coupling**

(71) Applicant: Steinbeis-Transferzentrum für Biopolymeranalytik und Biomolekül-Massenspektrometrie, 78464 Konstanz (DE)
(72) Inventor: PRZYBYLSKI, Michael, 65468 Trebur (DE); SLAMNOIU, Stefan, 78465 Konstanz (DE); MOISE, Adrian, 78464 Konstanz (DE)
(74) Representative: Müller-Boré & Partner Patentanwälte PartG mbB

(57) **Abstract**

The present invention relates to a microfluidic interface for the coupling of a bioaffinity sensor system to a mass spectrometry system, as well as to a respective method of using said interface.

## Description

The present invention relates to a microfluidic interface for the coupling of a bioaffinity sensor system to a mass spectrometry system, as well as to a respective method of using said interface.

Bioaffinity interactions play a key role in all mechanisms of cellular life. Among a variety of methods, biosensors have recently emerged as powerful tools for the detection and quantification of biomolecular interactions, and have been employed in a number of studies, such as protein-peptide, protein-polynucleotide and antigen-antibody interactions.

A general limitation of biosensors is the lack of chemical structure information of affinity-bound ligands. The chemical identification of ligands involved in biomolecular interactions, however, is of crucial importance for understanding interaction mechanisms in biochemical and biomedical processes, and in applications such as biomarker development and determination of lead structures for drug development. Hence, an interface for online bioaffinity-mass spectrometry coupling, would enable a broad application range for the combined affinity detection and quantification, and the mass spectrometric chemical structure analysis of affinity-separated biomolecular ligands.

Accordingly, the technical problem underlying the present invention is to provide a microfluidic interface for the coupling of a bioaffinity sensor system to a mass spectrometry system.

The solution to the above technical problem is achieved by the embodiments characterized in the claims.

In particular, in a first aspect, the present invention relates to a microfluidic interface for the coupling of a bioaffinity sensor system to a mass spectrometry system, said interface comprising:
(a) an affinity enrichment component (2), comprising:
   (i) one or more microchips (2a, 2b) for affinity enrichment; and/or
   (ii) microbeads for affinity enrichment;
(b) a transfer component, comprising;
   (i) a first 6-port low pressure switching valve (3a);
   (ii) a microcolumn for the desalting of samples (4); and
   (iii) a second 6-port low pressure switching valve (3b);
   and
(c) a solvent delivery component, comprising:
   (i) a pump system (6); and
   (ii) a solvent selection valve (5).

Preferably, in the microfluidic interface of the present application,
(1) the bioaffinity sensor system can be brought into fluid communication with the affinity enrichment component (2) via a capillary (1);
(2) the transfer component can be brought into fluid communication with the mass spectrometry system via a capillary (8);
(3) the affinity enrichment component (2) is fluidly connected to the first 6-port low pressure switching valve (3a);
(4) the affinity enrichment component (2) can be brought into fluid communication with a waste reservoir or with the microcolumn for the desalting of samples (4) via the first 6-port low pressure switching valve (3a);
(5) the microcolumn for the desalting of samples (4) is fluidly connected to the second 6-port low pressure switching valve (3b);
(6) the microcolumn for the desalting of samples (4) can be brought into fluid communication with a waste reservoir or with the mass spectrometry system via the second 6-port low pressure switching valve (3b);
(7) the solvent delivery component is fluidly connected to the first 6-port low pressure switching valve (3a);
(8) the solvent delivery system provides selection between different solvents (7) via the solvent selection valve (5); and
(9) the solvent delivery system can be brought into fluid communication with a waste reservoir or with the microcolumn for the desalting of samples (4) via the first 6-port low pressure switching valve (3a).

According to the present invention, the bioaffinity sensor system and the mass spectrometry system that can be coupled with said interface are not particularly limited. Suitable bioaffinity sensor systems include biosensors, selected from the group, consisting of surface plasmon resonance (SPR) biosensors, quartz crystal microbalance (QCM) biosensors, bio-layer interferometry (BLI) biosensors, surface acoustic wave (SAW) biosensors, nanopore optical interferometry biosensors, enzyme-based biosensors, and immunosensors. Further, suitable mass spectrometry systems can be selected from the group, consisting of electrospray ionization (ESI) mass spectrometry systems, matrix-assisted laser desorption-ionization (MALDI) mass spectrometry systems, desorption-electrospray (DESI) mass spectrometry systems, Fourier transform ion cyclotron resonance (FTICR) mass spectrometry systems, quadrupole time-of-flight (QTOF) mass spectrometry systems, linear ion trap mass spectrometry systems, orbitrap mass spectrometry systems, and generally all laser-ionization mass spectrometry systems and spray-ionization mass spectrometry systems. Respective bioaffinity sensor systems and mass spectrometry systems, as well as further bioaffinity sensor systems and mass spectrometry systems that can be used in connection with the microfluidic interface of the present invention, are known in the art.

The affinity enrichment component (2) of the microfluidic interface of the present invention provides enrichment of the analyte that has been analyzed in the bioaffinity sensor system. Enrichment is effected by binding to the surface of one or more microchips for affinity enrichment and/or to the surface of microbeads for affinity enrichment. Suitable microchips and microbeads are not particularly limited and are known in the art for any given analyte. In particular embodiments, the one or more microchips for affinity enrichment are selected from the group consisting of gold-coated affinity chips and affinity chips having a chemically modified surface. In case gold-coated affinity chips are used, wherein the surface of the gold-coated affinity chips is preferably coated with a self-assembled monolayer (SAM) of 16-mercaptohexadecanoic acid or 11-mercaptoundecanoic acid for the coupling of polypeptides via covalent attachment of amino functions to said 16-mercaptohexadecanoic acid or 11-mercaptoundecanoic. In this embodiment, the free carboxyl groups of 16-mercaptohexadecanoic acid or 11-mercaptoundecanoic are activated by a mixture of N-hydroxysuccinimide and 1-ethyl-3-(3-dimethylaminopropyl)-carbodiimide (EDC). After activation, the active ester component reacts with free amino groups of the polypeptide, thus forming amide bonds. In case affinity chips having a chemically modified surface are used, the chemical modification is preferably attachment of long-chain alkyl-thiol groups for the coupling of polypeptides via covalent attachment of amino functions to said alkyl-thiol groups. In a particular embodiment of the present invention, the affinity enrichment component (2) of the microfluidic interface of the present invention comprises two microchips, e.g. microchips for affinity enrichment as defined above.

The interface may include and/or may be built from any OEM (Original Equipment Manufacturer) and consumer electronic components or devices with digital and/or analog inputs and outputs including but not limited to: gas and liquid pumps and compressors, valves, pressure sensors, flow sensors, IR sensors, relays, various motors (AC, DC motors with or without quadrature encoders, stepper motors), current and voltage converters, A/D converters, various communication modules, microcontroller assemblies and various embedded systems.

The interface and PC can send and receive information to and from the components via communication interfaces including but not limited to the following: RS232, RS485, TCP/IP, CAN, 12C, SPI, USB, TTL. Radio an telecommunication interfaces, devices and components based on GSM, Bluetooth, IR, WIFI may also be included.

The transfer component of the microfluidic interface of the present invention provides a first and a second 6-port low pressure switching valve (3a, 3b), as well as a microcolumn for the desalting of samples (4). Suitable switching valves are not particularly limited and are known in the art. They include e.g. switching valves that are manufactured and marketed under the name Rheodyne™. Suitable microcolumns for the desalting of samples are not particularly limited and are known in the art. Respective microcolumns are sometimes referred to herein as guard columns. Suitable column materials are not particularly limited and are known in the art for matching any given analyte. Such materials include for example C4 or C18 columns. Column materials could be in particular, reversed phase materials, such as long chain-alkyl, amino-alkyl, cyano-alkyl; cation-exchange materials; anion-exchange materials.

The solvent delivery component of the microfluidic interface of the present invention comprises a pump system (6) and a solvent selection valve (5), thus providing the possibility to stream various suitable solvents through the interface. The pump system preferably comprises a precision micro pump, which is preferably capable of providing flow rates in the range of 360 nl/min to 300 µl/min. Suitable pump systems and solvent selection valves are not particularly limited and are known in the art. Respective valves include e.g. valves that are manufactured and marketed under the name Rheodyne™.

Preferably the microfluidic interface of the present invention further comprises a control electronics system for
(1) controlling the operation of the first and second 6-port low pressure switching valves (3a, 3b);
(2) controlling the operation of the pump system (6) and the solvent selection valve (5);
(3) providing two-way communication with the bioaffinity sensor system;
(4) providing two-way communication with a computer; and/or
(5) providing two-way communication with the mass spectrometry system.

Thus, the interface of the present invention can provide means for receiving and sending information and/or instructions to/from the bioaffinity sensor system, a computer, and/or the mass spectrometry system. In a preferred embodiment, the control electronics system provides two-way communication with a computer, wherein the computer is running computer-readable instructions for controlling the functions of the interface. Preferably, the functions of the interface that are controlled by the computer include monitoring sample injection in the bioaffinity sensor system, detection of elution profile and sample flow, triggering of mass spectrometric data acquisition, monitoring elution injection, and switching valves for sample transfer. Preferably, the control electronics include a relay acting as electrical trigger for synchronization of sample delivery from the interface for the acquisition of mass spectra. Further, respective interface functions that are controlled by the computer may be in particular affinity-enrichment; isolation; proteolytic digestion; chemical modification; and related functions.

In a second aspect, the present invention relates to a method for the coupling of a bioaffinity sensor system to a mass spectrometry system, comprising the step of appropriately connecting the bioaffinity sensor system and the mass spectrometry system to the microfluidic interface of the present invention. In this aspect, the bioaffinity sensor system and the mass spectrometry system can be as defined above.

The present invention describes a microfluidic interface for the direct (online) biosensor-mass spectrometry coupling. The interface can e.g. be employed, and is described herein, for the online mass spectrometry combination of a surface acoustic wave (SAW) biosensor to electrospray ionization mass spectrometry (SAW-MS), and provides simultaneous affinity detection, mass spectrometric chemical structure determination, and quantitative determination of affinity (binding) constants of affinity-ligands such as polypeptides, carbohydrates and nucleotides. However, the interface for online biosensor-mass spectrometry according to the present invention is applicable with different biosensor systems based on different detection principles, e.g. surface plasmon resonance (SPR), quartz crystal microbalance (QCM), bio-layer interferometry (BLI) and surface acoustic wave (SAW); and with different mass spectrometric ionization systems, such as electrospray ionization (ESI), matrix-assisted laser desorption-ionization (MALDI) and desorption- electrospray (DESI).

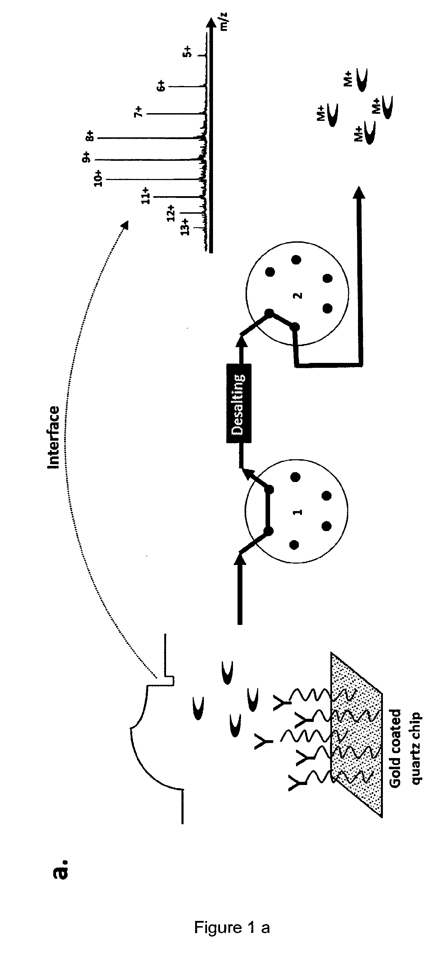

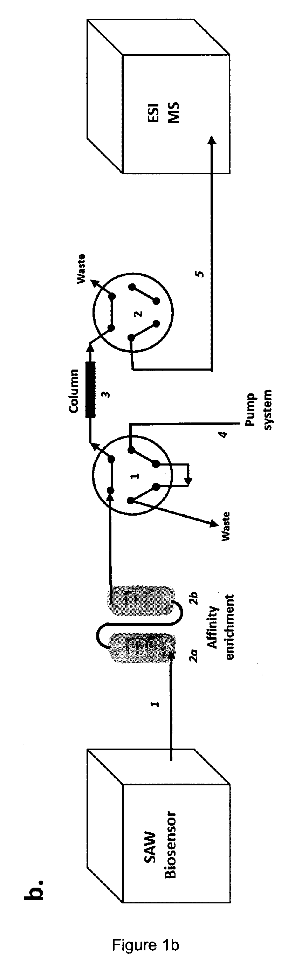

Key functions of the microfluidic interface of the present application are (i) affinity-enrichment, (ii) desalting of samples eluted from the biosensor and (iii), transfer of ligands into the ion source of the mass spectrometer. The microfluidic interface comprises a microchip (or alternatively microbeads) for affinity-enrichment, sample concentration, and a microcolumn for desalting of samples and transfer to MS analysis (Figure 1). Coupling of the interface and corresponding mass spectrometric analyses have been carried out, and are feasible, e.g. with a quadrupole ion trap-mass spectrometer, a high resolution Fourier transform Ion Cyclotron Resonance mass spectrometer (FTICR-MS), and a Quadrupol Time-of-Flight (QTOF) mass spectrometer, as shown in the examples of the present application.

However, applications using a wide range of other mass spectrometer types, e.g. linear ion trap and orbitrap mass spectrometers, are feasible. Describes herein is also suitable software for the MS-acquisition coupling of the interface to several MS systems.

Key element for the online biosensor-mass spectrometry (MS) combination according to the present invention is an interface that provides sample enrichment and isolation, *in situ* desalting and transfer of the ligand eluate to the MS system. A suitable microfluidic interface incorporating a desalting microcolumn was initially developed and applied for an ESI-ion trap mass spectrometer (such as Bruker Esquire-3000+ quadrupole ion trap-MS system). The automated coupling interface (Figure 1) used for online SAW-biosensor-MS is controlled through a specific software (SAW2MS) for (i) monitoring sample injection in the SAW biosensor, (ii) detection of elution profile and sample flow, and (iii) triggering the MS data acquisition.

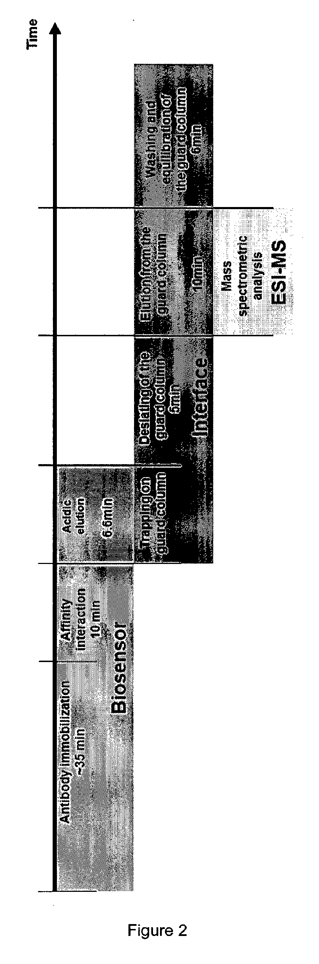

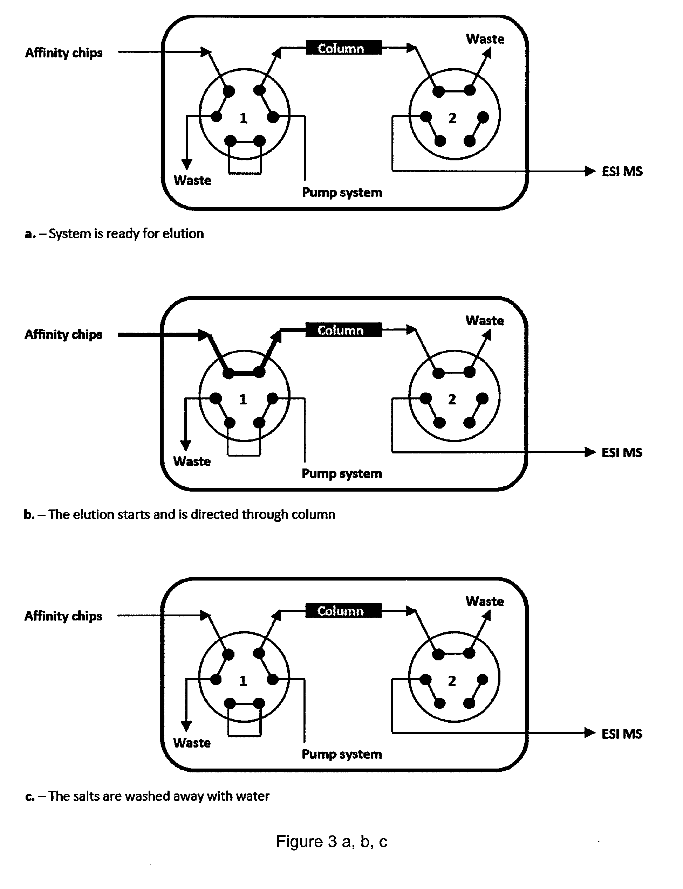

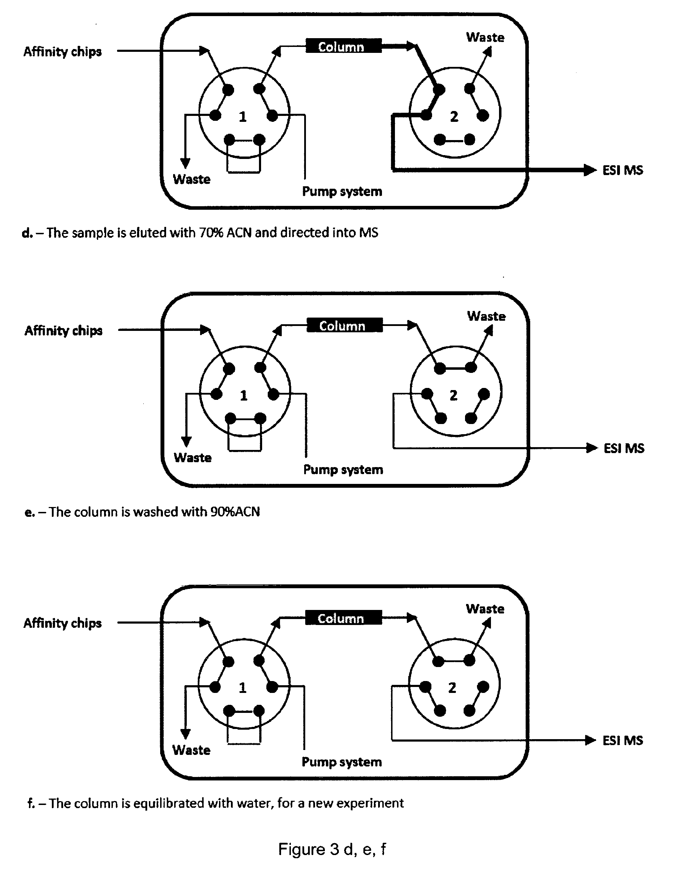

Since biosensors usually employ a microfluidic system with continuous flow, coupling to an ion source for MS is straightforward. However, the high-salt PBS buffer concentration used in biosensor binding experiments at physiological solution conditions would present major problems for MS. Moreover, typical biosensor flow rates are not compatible with the standard solvent flow for MS. These problems are solved by the interface of the present invention which simultaneously provides (i) desalting by a suitable MS-compatible solvent mixture; (ii) sample concentration by suitable solvent change for release of affinity-ligands; and (iii) automated flow rate equilibration between the biosensor (e.g. approximately 15 µl/min) and the ion source (e.g. approximately 30 µl/min). Details of the time sequence of the interface operation, and operation sequence of the switching valves are described in Figures 2 and 3. The BioSensor2MS software was used for monitoring the elution injection, switching the valves for sample transfer and triggering the MS acquisition once the sample is sent to the ESI source. The software was developed using Visual Basic (Visual Studio 2010) and is compatible with version 4.0 of the .NET framework.

An analogous interface to that described for transfer of the SAW-biosensor eluate into the ion source of an electrospray-MS system has been developed for transfer to (i) a MALDI-MS system; and (ii) a desorption-electrospray ionization system. In this combination, the eluate is transferred directly onto the sample application stage, or multiwell target of a MALDI ion source; the fractions specified by the time intervals selected in the interface control (Figure 2) can be analyzed sequentially by MALDI-MS.

The performance of the interface was initially tested by characterization of the interaction of the model system calmodulin-melittin, a calcium receptor involved in a variety of physiological affinity-binding processes. Immobilization of calmodulin and its interaction with melittin were analyzed by K_{D} determination and by mass spectrometric identification of the eluted peptide ligand. Examples of bioanalytical application to antibody- polypeptide ligands, and to analysis from biological material are described in the following sections.

Bioanalytical applications of the interface of the present application for online SAW-biosensor-MS are shown hereinafter for affinity quantifications and mass spectrometric identifications of the neurodegenerative polypeptides, β-amyloid (Aß), and α-Synuclein (αSyn). Moreover, the efficacy of the online affinity (SAW)-MS combination is demonstrated by the direct analysis of αSyn from biological material, such as brain tissue homogenate, illustrating the broad application potential of the online affinity-MS interface of the present invention for identification, quantitative affinity determination and structural characterization of ligands in biopolymer interactions.

The figures show:
Figure 1:
   Scheme of the online SAW - MS interface.
   (a) Analytical concept showing the interface of the present invention as an integrating microfluidic component between a SAW biosensor and mass spectrometer (MS). The affinity-isolated, eluted analytes are transferred to the MS via the microfluidic interface. (b) Technical scheme showing the interface components. The SAW chip is connected to the interface by a fused silica capillary (1). An affinity-enrichment chip is placed into the microfluidic system by a silicone cell (2). The analyte is sent via two automatic 6-port low pressure switching valves (Rheodyne) to a guard micro-column (3) providing buffer desalting, flow rate equilibration, and transfer of eluate to the ESI source (5). A precision micropump (e.g., µHPLC pump) (4) is used for transfer of solute through the desalting column. Interface, sample transfer and MS acquisition are operated by the Biosensor2MS software.
Figure 2:
   Event time line of SAW-MS interface.
   Three horizontal bars along the time axis show the operation time periods of SAW-MS interface and transfer to MS: Biosensor, point pattern; interface, diagonal line pattern; transfer and mass spectrometer, cross-line pattern. Perpendicular lines on the time axis delineate the different operation steps. The stages of operation are described in Figure 3.
Figure 3:
   Stages of interface operation during online affinity-MS.
   The positions of electrical valves and sample path through the interface are as follows: (a) Interface is monitoring the SAW biosensor for the elution injection; incoming flow from biosensor is sent to waste, while the desalting column is rinsed with solvent. Valve 1 is in position (i) and valve 2 in position (ii). (b) The elution injection is detected, and the sample flow from the biosensor is directed to the guard column; valve 1 is in position (ii) and valve 2 is in position (i). (c) following completion of the elution injection, salts contained in the sample are washed out from the column; valve 1 is in position (i) and valve 2 is in position (ii). (d) The sample trapped on the guard column is eluted with a high acetonitrile-containing solvent and transferred to the ESI source; valve 1 is in position (i) and valve 2 is in position (i). (e) The guard column is cleaned of remaining contamination with a solvent containing 90 % acetonitrile, which is sent to waste; valve 1 is in position (i) and valve 2 is in position (ii). (f) After cleaning, the guard column is equilibrated with water, being set ready for a new experiment; valve 1 is in position (i) and valve 2 is in position (ii).
Figure 4:
   Identification and affinity quantification of an Aβ-epitope specific antibody-Aβ(1-40) complex by online SAW-FTICR-MS.
   (a) SAW binding curve and affinity interaction of the immobilized Aβ-antibody (4G8) with pre-aggregated Aβ(1-40); (b) Total ion Current (TIC) of the biosensor eluate transferred to the ion source; (c) ESI-FTICR mass spectrum of the eluted Aβ; (d). K_{D} determination of the Aβ-antibody complex. Mass determination accuracy of Aβ(1-40) was approximately 6 ppm.
Figure 5:
   Identification and affinity quantification of an Aβ-epitope specific antibody-Aβ(12-40) complex by online SAW-MS, using an Synapt-HDMS-QTOF instrument.
   (a) SAW binding curve and affinity interaction of the immobilized Aβ-antibody (4G8) with Aβ(12-40). (b) TIC of the biosensor eluate transferred to the ion source; (c) ESI mass spectrum of the eluted Aβ(12-40); (d) K_{D} determination of the Aβ-antibody complex.
Figure 6:
   Comparison of affinity binding and mass spectrometric identification of αSyn and βSyn.
   (a) SAW binding curve of αSyn to the immobilized anti-αSyn pC20 antibody and ESI-ion trap mass spectrum of the eluted αSyn fraction. (b) SAW binding curve of βSyn to the anti-αSyn pC20 antibody and ESI-ion trap mass spectrum of the eluted βSyn fraction.
Figure 7:
   Online SAW-MS identification of the human αSyn(A30P) mutant from mouse brain homogenate.
   (a) SAW binding curve of αSyn from mouse brain homogenate to immobilized anti-αSyn antibody. (b) TIC profile of the eluate from mouse brain homogenate, sent to ESI ion trap MS. (c) ESI-ion trap mass spectrum of the eluate from mouse brain homogenate, showing 13+ to 23+ charged molecular ions.
Figure 8:
   Online FTICR-MS analysis of α-synuclein, mutant αSyn(A30P).
   (a) SAW binding and elution curve from immobilized anti-αSyn-antibody; (b) TIC of the biosensor eluate transferred to the ion source; (c) ESI-FTICR-MS of the eluted αSyn(A30P). (b) Online SAW-MS identification of the human αSyn(A30P) mutant from mouse brain homogenate. (Top-to-bottom): SAW binding curve of mouse brain homogenate to immobilized anti-αSyn antibody; TIC of the affinity-eluate; FTICR-MS analysis of the eluate from mouse brain homogenate; gel electrophoresis and Western blot of the mouse brain homogenate sample.
Figure 9:
   Schematics of a microfluidic interface according to the present invention.
   The SAW biosensor exit capillary (1) is connected to the chip component (2) which consists of microfluidic (2a and 2b) units covering gold coated chips, fixed in a metallic frame. The chip component is further connected to a set of two Rheodyne valves (3a and 3b), that encompass the desalting column (4). The solvent delivery system is composed of a pump (6) and a solvent selection valve (5), and is also connected to the first valve (3a). The solvent delivery provides the selection between several solvents (7) for sample desalting, elution into the MS, and column cleaning and re-equilibration. The second valve (3b) is connected to the ESI ion source of the mass spectrometer through the capillary (8).
Figure 10:
   SAW-Affinity Interface housing.
   Based on the mechanical setting of the biosensor, the flow-direction goes from right to left. Accordingly, the first valve (IN-valve) is the right valve.
Figure 11:
   Affinity enrichment component of an interface according to the present invention with closed and open flap.
Figure 12:
   Solvent delivery system (front) of an interface according to the present invention.
Figure 13:
   Solvent delivery system (back) of an interface according to the present invention, showing the cable connections.
Figure 14:
   Transfer component of an interface according to the present invention.
Figure 15:
   Tubing connections of an interface according to the present invention.

The present invention will be further illustrated in the following examples without being limited thereto.

### Examples

### Example 1:

### Characterization of interactions of Aβ-polypeptides and anti-Aβ antibodies by online SAW-MS

Examples of applications of the online SAW-MS combination are described in the following for the affinity characterization of antibodies specific for β-amyloid (Aβ), the key polypeptide in Alzheimer's disease (AD), and several other key proteins for neurodegenerative disorders (Table 1, below). The detailed biochemical pathways and mechanism(s) underlying AD are still unclear; however, extracellular plaques containing aggregated Aβ-peptide have been shown to play a major role, and Aβ-oligomers have been identified to exert major neurotoxicity effects. Hence, Aβ-specific antibodies capable of disaggregating Aβ-plaques or inhibiting Aβ-aggregation are gaining increasing interest for potential AD immunotherapy.

Epitope-specific anti-β-amyloid (Aβ)-antibodies were immobilized on a gold chip surface using a 16-mercaptohexadecanoic acid silyl-based alkyne modifying (SAM) linker, and Aβ(1-40) and Aβ(12-40) peptides, respectively.

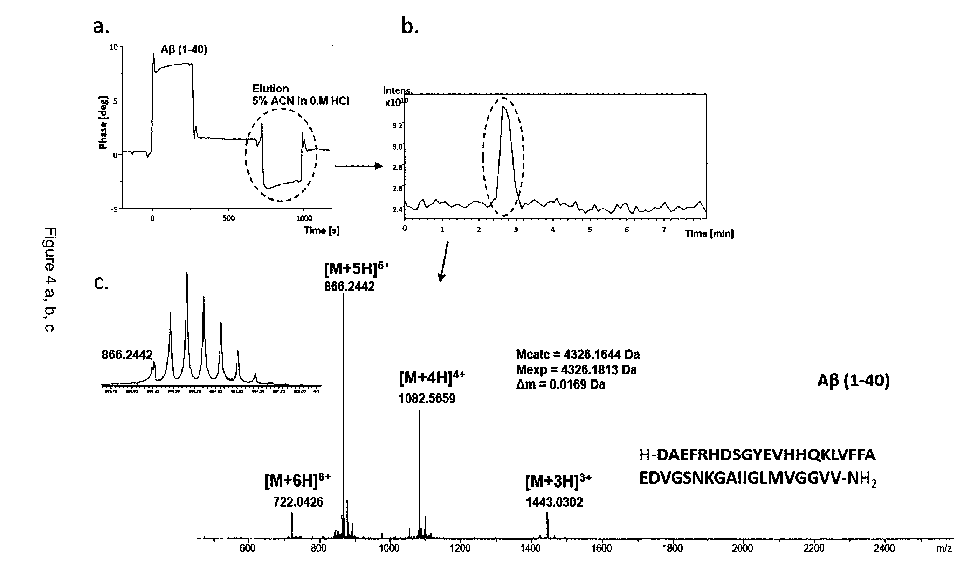

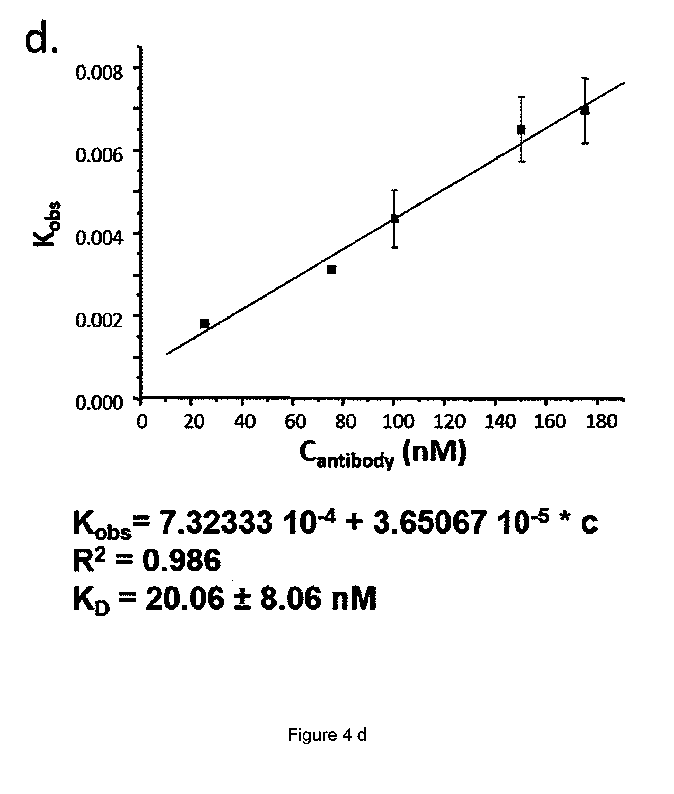

Epitope-specific Aβ-antibodies were studied by online-SAW-MS for characterization of Aβ-peptides and quantification of affinities. The Aβ-epitope recognized by the antibodies have been identified in previous studies by proteolytic excision-mass spectrometry (Table 1, below). The mass spectrometric analysis of the epitope specificity revealed that the aggregation-inhibiting antibody (4G8) binds to a central to C-terminal epitope, Aβ(17-24). In contrast, an N-terminal epitope, Aβ(4-10), is recognized by a plaque-disaggregating antibody (6E10). The online-SAW-MS analysis of a pre-aggregated Aβ(1-40) sample from an anti-Aβ-antibody (4G8) capable of inhibiting Aβ-aggregate formation is shown in Figure 4. Upon interaction with the antibody immobilized on the chip surface and elution from the interface, Aβ(1-40) was identified by the mass spectrometric analysis (Figure 4, a to c). From the affinity binding curve, a dissociation constant K_{D} of approximately 20 nM was determined (Figure 4 d). For comparison, the online- SAW-MS analysis of Aβ(4-10) ascertained that the N-terminal peptide alone had no affinity to the antibody. Binding affinities determined by SAW-MS agreed well with previous affinity determinations using conventional methods. No significant change of elution and peak profile was observed in at least 5 repeated MS analyses and affinity quantifications of Aβ(1-40), upon elution from the antibody; no significant increase of background signal was observed upon regeneration of the chip surface.

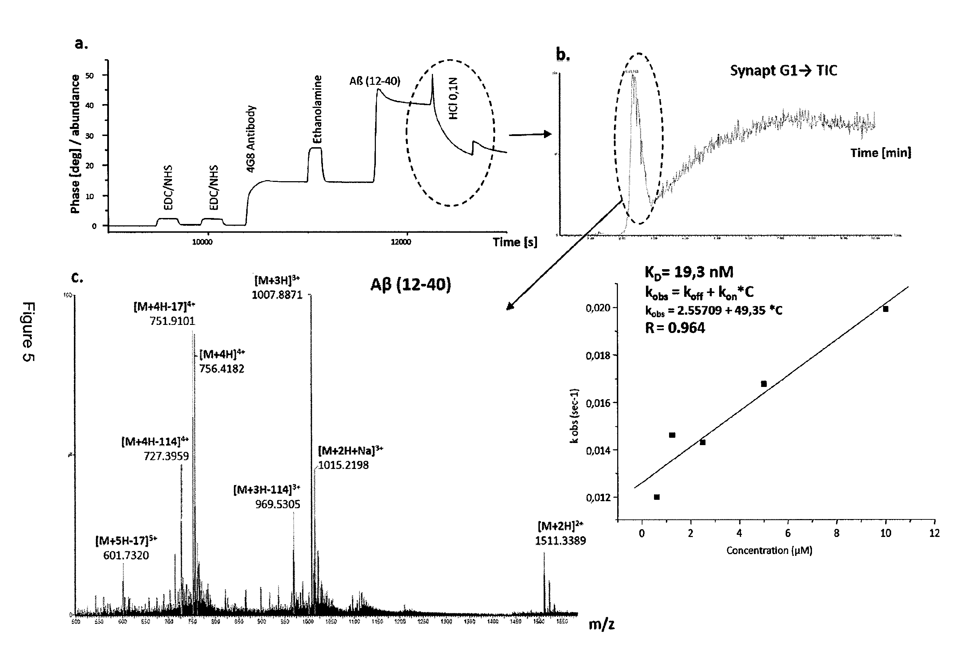

The identification and affinity quantification of the Ap- antibody (4G8) complex with Aβ(12-40) peptide by online SAW-ESI-MS coupling to a ESI-QTOF mass spectrometer is shown in Figure 5. Both the SAW binding curve and affinity elution profile from the immobilized Aβ-antibody were very similar to the SAW-MS analysis in Figure 4; the ESI spectrum provided unequivocal identification of Aβ(12-40) (Figure 4 c). The affinity determination provided a K_{D} of 19.3 nM, which is nearly identical with the K_{D} of 20.1 nM found for Aβ(1-40) (Figure 4), and in agreement with the epitope specificity of the 4G8 antibody in the C-terminal sequence of Aβ. Thus, the online SAW-affinity-MS interface can be adapted to different ESI-MS systems.

### Example 2:

### Identification and affinity characterization of α-Synuclein from brain homogenate

Aggregation of the key protein of Parkinson's disease, α-Synuclein (αSyn), is thought to proceed via oligomers of high neurotoxicity. Studies of the *in vitro* oligomerization-aggregation recently provided the first identification of specific autoproteolytic degradation products; particularly, a highly aggregation-prone C-terminal fragment, αSyn(72-140), was identified upon incubation of αSyn for several days at physiological pH 7. In contrast, β-synuclein (βSyn), the non-aggregating, non-toxic synuclein polypeptide in brain with a mutant VFS(70-72) sequence that lacks the cleavage site VVT(70-72) in the central amyloidogenic domain, showed neither oligomerization-aggregation nor any autoproteolytic cleavage. These results afforded high interest in the comparative molecular characterization of αSyn and βSyn polypeptides *in vivo* by affinity-mass spectrometry using synuclein-specific antibodies.

Epitope-specific anti-α-Synuclein-antibodies were immobilized on a gold chip surface using a 16-mercaptohexadecanoic acid SAM linker. Synuclein polypeptides βSyn, αSyn and overexpressed αSyn(A30P) mouse brain homogenate were eluted at acidic conditions (pH 2.5) for MS analysis.

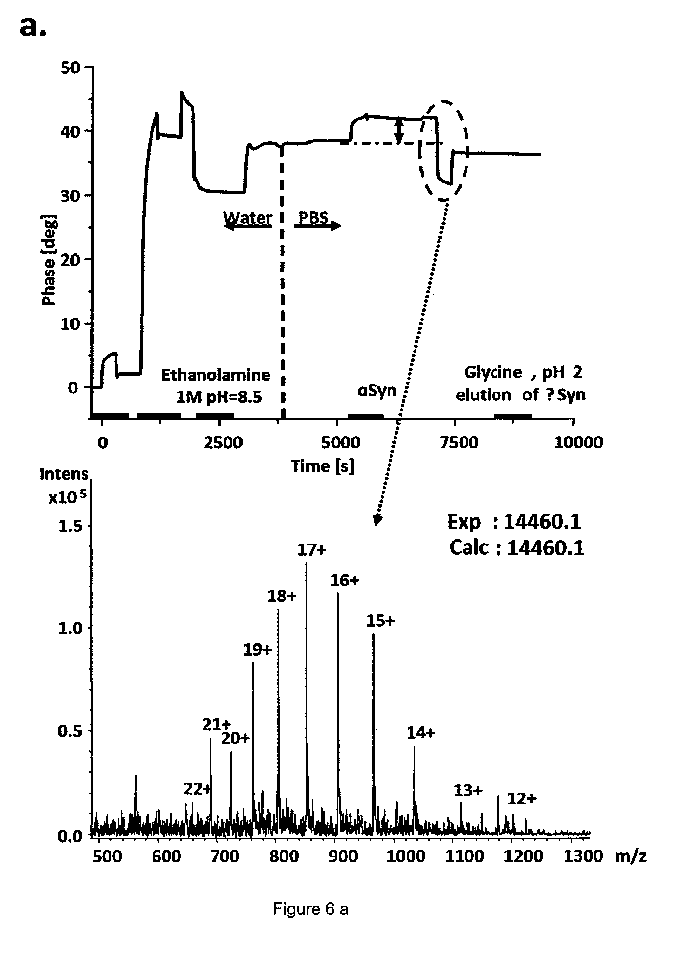

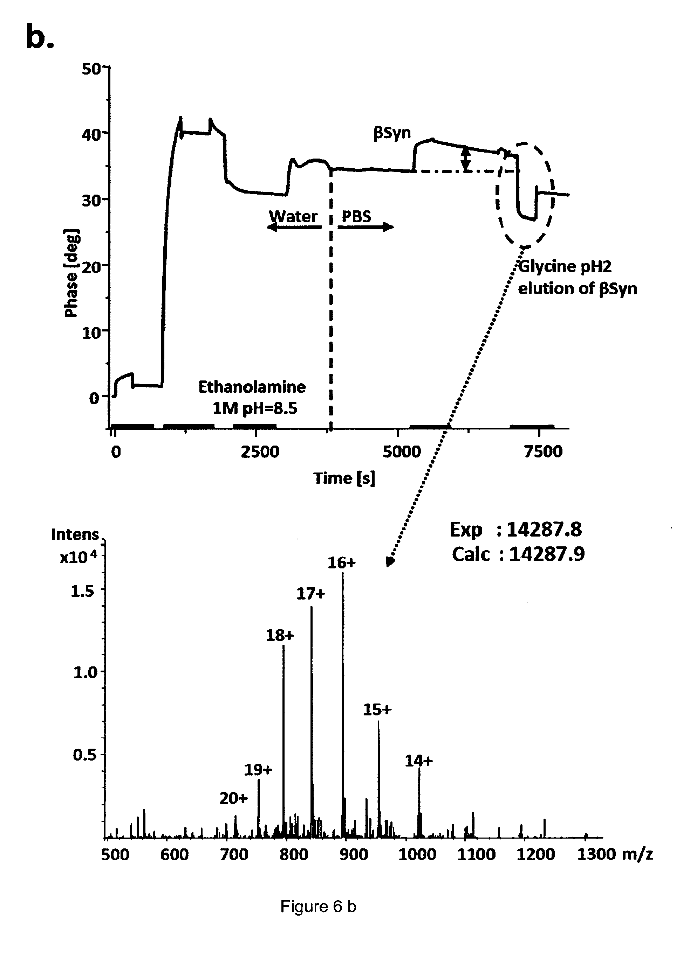

Figure 6 shows the comparison of affinity binding and mass spectrometric identification of αSyn and βSyn by online affinity-MS, using an immobilized antiαSyn antibody (pC20; Table 1). The SAW binding curves were similar for both proteins; the ESI-ion trap mass spectra of the eluted polypeptides provided unequivocal identifications. The affinity determinations yielded similar K_{D} values of approximately 40 nM for both polypeptides, in agreement with the epitope specificity of the synuclein-antibody in the C-terminal domain Syn (81-96) which is identical for both polypeptides (Table 1).

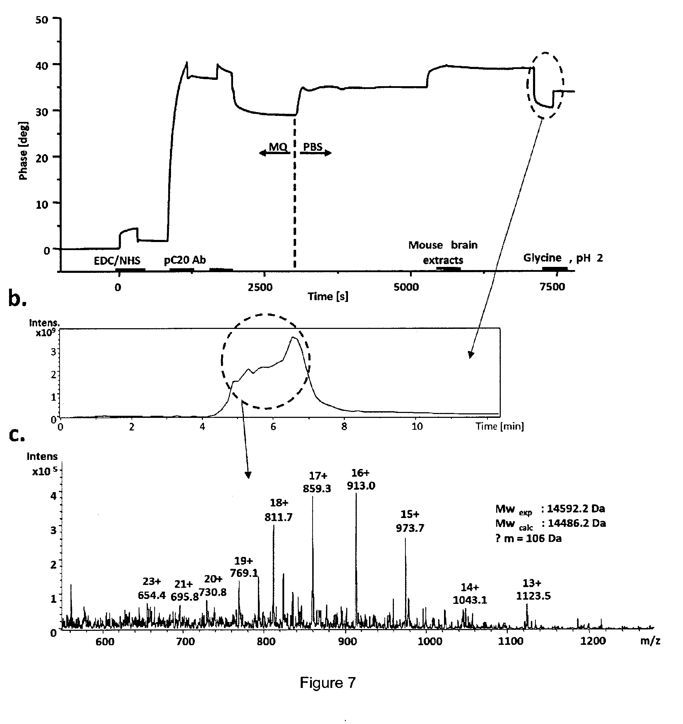

First applications to synucleins in brain tissue were performed in order to evaluate the feasibility of online SAW-MS to the direct analysis of biological material. Using a chip-immobilized antibody recognizing a C-terminal αSyn epitope (4B12; Table 1), direct identifications of α-synuclein and αSyn oligomers were obtained from brain tissue. Figure 7 shows the SAW binding curve and online SAW-ESI-MS analysis of the interaction of mouse brain homogenate expressing the human αSyn(A30P) mutant with the anti-αSyn antibody 4B12. A dissociation constant K_{D} of approximately 45 nM was determined for the αSyn-antibody complex. The mass spectrometric analysis revealed a multiply charged ion series with a molecular mass of 14486 Da, in close agreement with the expected molecular weight of the αSyn mutant (Figure 7 a to c).

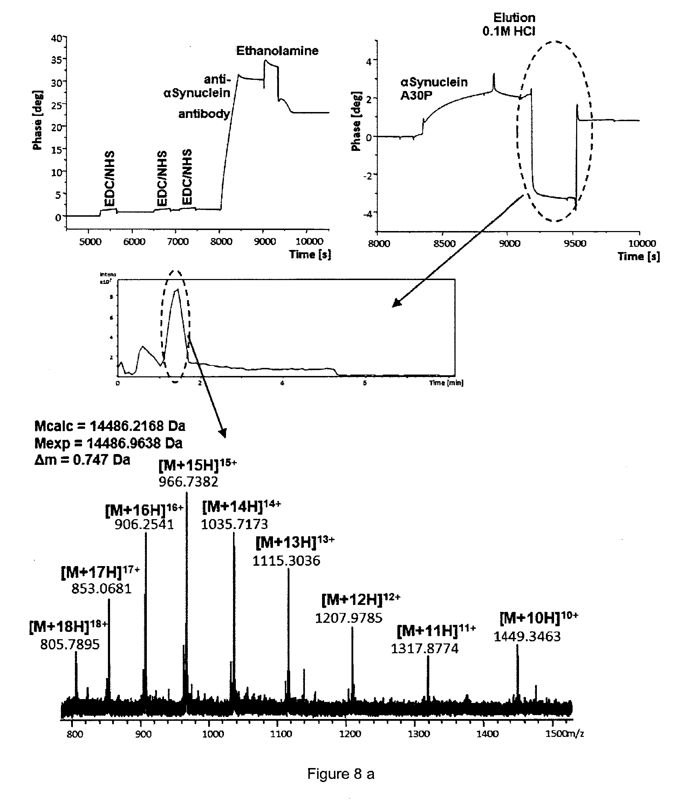

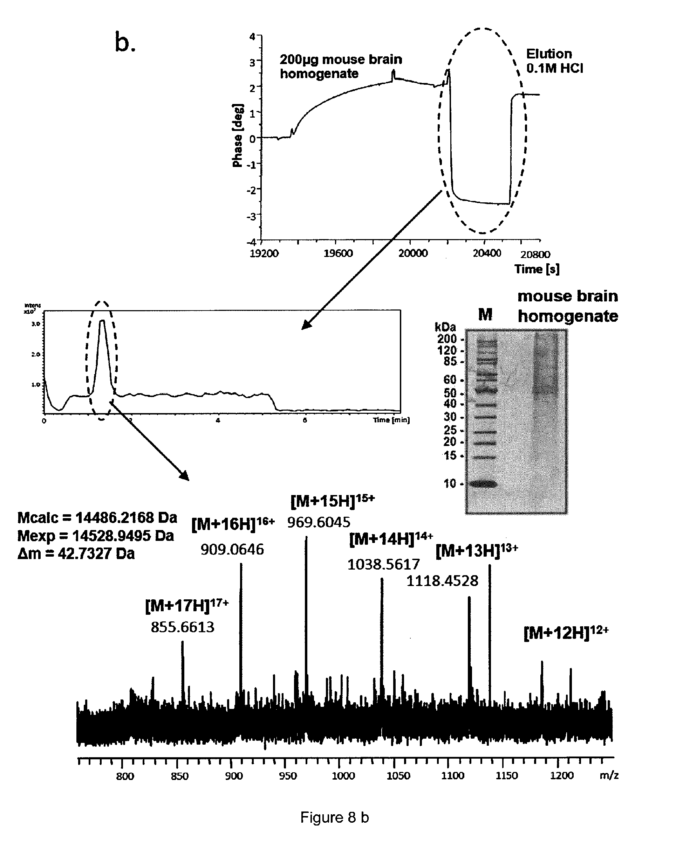

A comparison of the analysis of αSyn(A30P) by online SAW-FTICR-MS *in vitro* and from mouse brain homogenate is shown in Figure 8. The SAW binding curve of the immobilized anti-αSyn-antibody, and high resolution ESI-FTICR mass spectrum of the affinity-eluted αSyn(A30P) provided a molecular mass of 14486 Da, consistent with the intact protein. In the online SAW-MS analysis from mouse brain homogenate, a very similar binding curve and elution profile was obtained. The FTICR-MS analysis of the eluate from brain homogenate yielded a molecular mass of 14528 Da, corresponding to a mass increase by 42 amu compared to the mass of 14486 Da for unmodified αSyn(A30P) determined *in vitro.* This modification was identified by subsequent sequence determination as posttranslational acetylation. Using the same antibody, a comparative Western blot of the brain sample indicated αSyn(A30P) monomer as a major product together with low levels of possible oligomer and truncation products, however no possible identification is amenable; for comparison a standard electrophoresis did not indicate the presence of synuclein or related proteins (Figure 8 d, e).

**Table 1: Epitope analysis and K_{D} determination of neurodegenerative proteins using specific antibodies.**

| **Protein/ Polypeptide** | **MW (Da)** | **Antibody** | **Epitope Sequence** | **K_{D} (10⁻⁹ M)** |
|---|---|---|---|---|
| Aβ(1-40) | 4326.1 | Anti Aβ (17-24) [4G8]^{a} | (17-24) | 20.1 ± 8 |
| Aβ(1-40) | 4326.1 | Anti Aβ (1-16) [6E10]^{a} | (4-10) | 16.3 ± 5 |
| α-Synuclein | 14486.2 | Anti α-Synuclein [4B12]^{b} | (81-96) | 45.5 ± 3 |
| α-Synuclein | 14486.2 | Anti-αSyn pC20 | (81-96) | 39 ± 11.4 |
| h Tau (4R/2N) | 45849.9 | Anti hTau [TAU5]^{a} | (210-230) | 48.9 ± 12 |

| | | | | |
|---|---|---|---|---|
| ^{a} Monoclonal Ap- antibody [4G8], Covance Inc., Princeton, USA. ^{b} Monoclonal αSyn antibody [4B12], GeneTex, Irvine, USA. | | | | |

### Example 3:

### Detailed technical description of an interface according to the present invention

The following provides a detailed description of a microfluidic interface of the present invention. Although this description relates to a specific interface for coupling SAW biosensors to ESI mass spectrometry systems, the embodiments describes hereinafter also apply to interfaces for coupling different bioaffinity sensor systems to different mass spectrometry systems according to the present invention. Further, said embodiments also apply generally to the microfluidic interfaces of the present invention.

### Overview of the system components

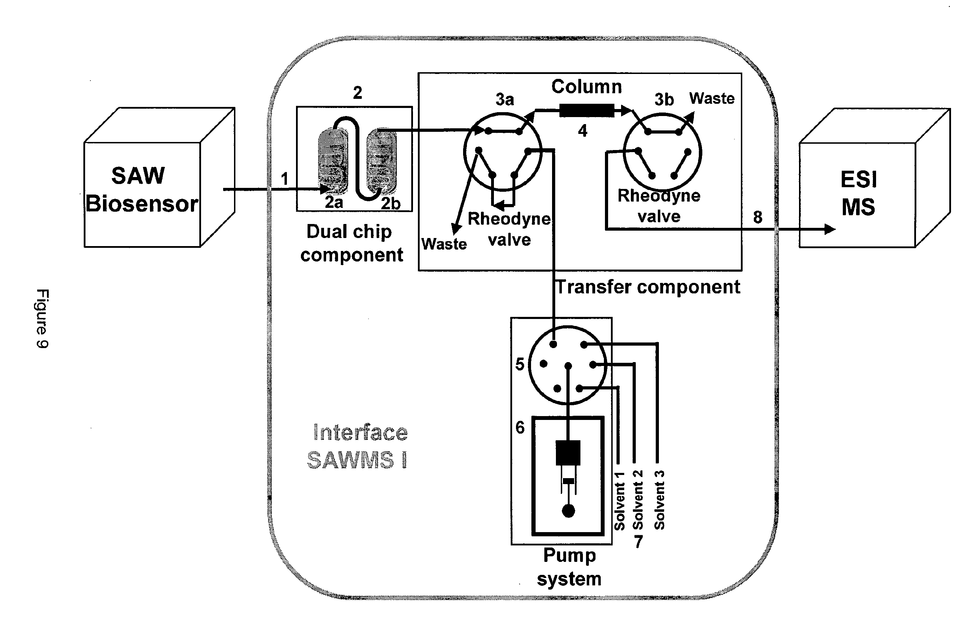

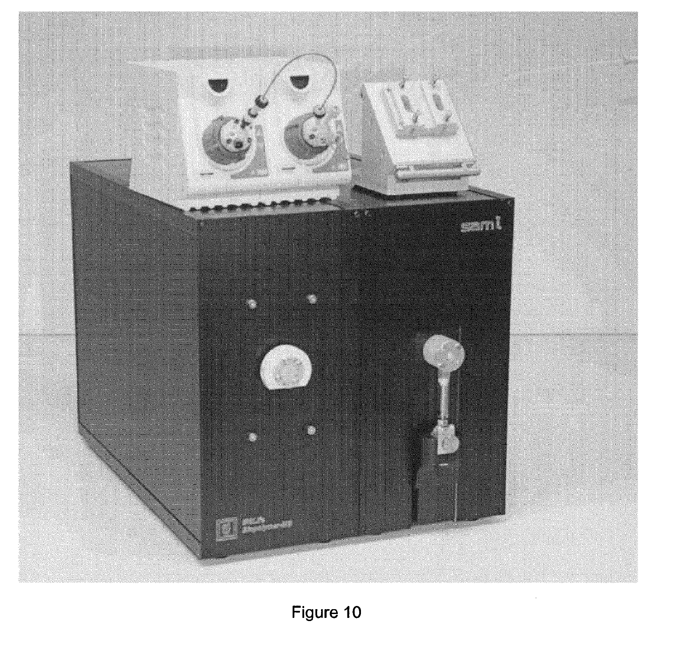

The interface SAW-MSLife-1 consists of hardware and software components. The hardware components are shown in Figure 9 which gives a schematic overview of the complete system.

### Affinity- enrichment chip component

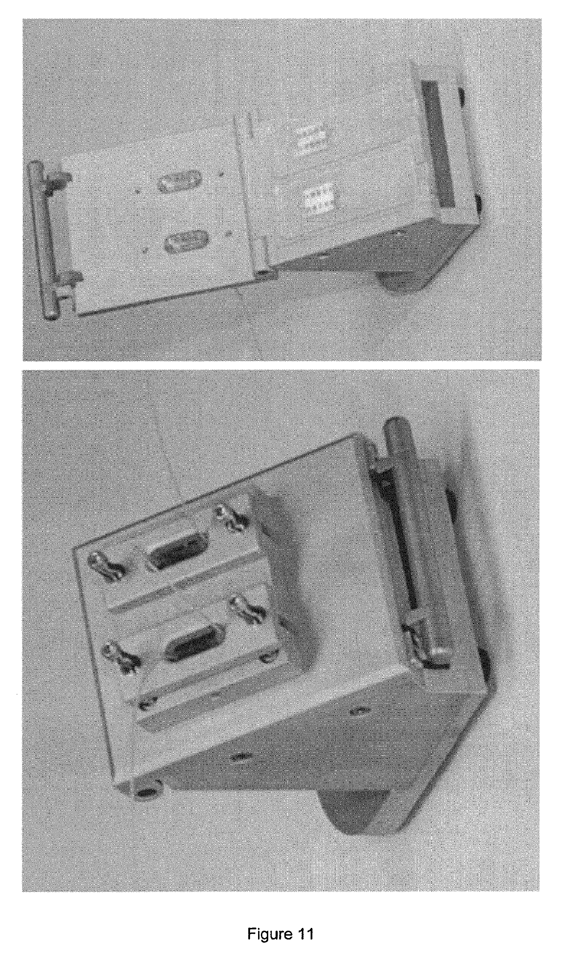

The amount of affinity-isolated sample for mass spectrometry analysis is generally increased by extending the binding surface. This is performed by the use of a gold coated affinity-chip (additionally to the one used for biosensor detection), or alternatively affinity-beads which are used for independent analyte affinity-enrichment (Affinity-Selector). The chip is typically prepared with a gold surface; However, a large number of specific chemical modifications of the surface can be employed, such as attachment of long-chain alkyl-thiol groups for covalent attachment of amino-functions, for coupling of polypeptides. The microfluidic system of the chip component can be opened to allow handling of the chip. When running an experiment using the chip component, the system is leak-proof closed (Figure 11).

### Solvent Delivery System

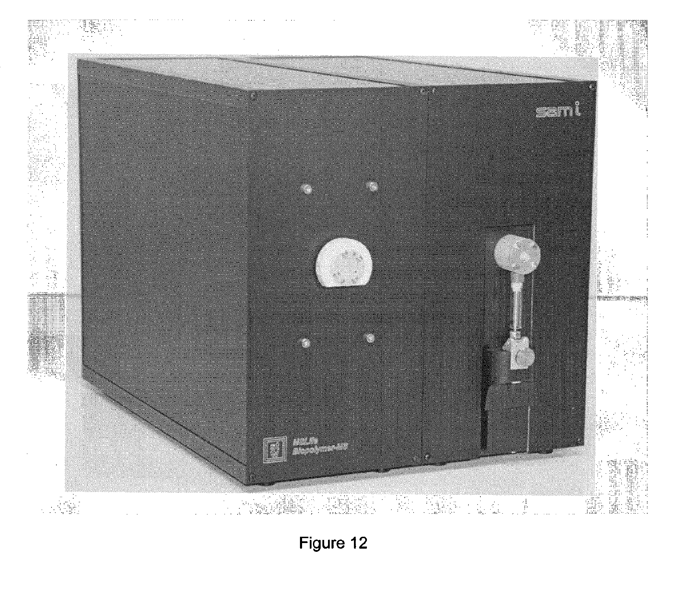

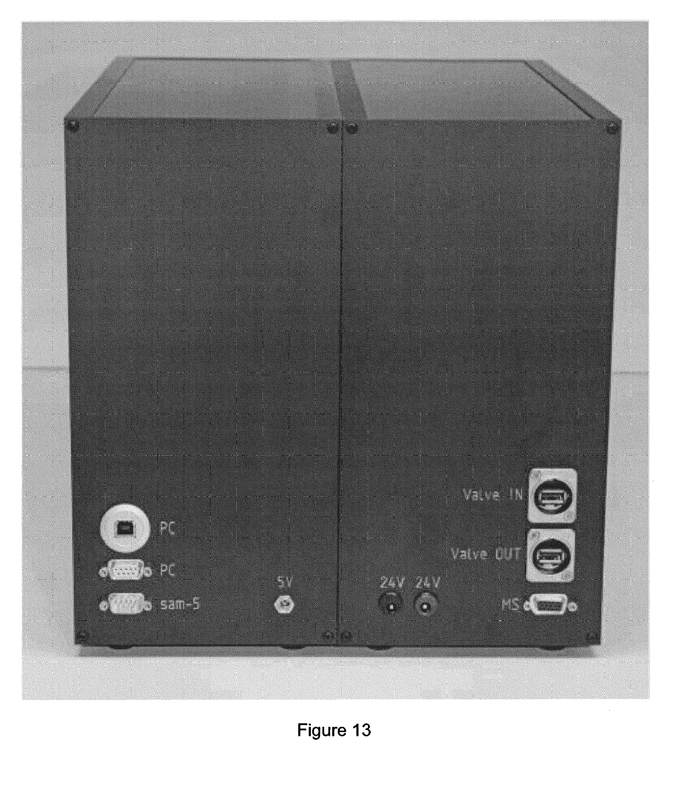

The solvent delivery system (Figure 12) consists of a precision micro pump and a Smart-Valve for solvent selection. The solvent delivery system is embedded in a metallic box together with the control electronics including a relay acting as electrical trigger for synchronization of sample delivery from the Interface for the acquisition of mass spectra. The solvent delivery system is capable to supply flow rates between approximately 360 nl/min and 300 µl/min. It typically operates at a flow rate of ∼20 µl/min, depending on the flow conditions of the mass spectrometer. The flow rate is adjusted to be compatible with the optimal flow rate of the biosensor. However, the flow rate of the MS-interface can be increased up to 100 µl/min.

The solvent delivery system is connected through cables to the other interface components, to the biosensor and the mass spectrometer as follows:
The "Valve IN" and "Valve OUT" are connected to the two valves of the transfer component via USB cables.
A serial connection is connected to the biosensor instrument through a serial cable.
The biosensor computer is connected to the USB connection "PC" and a serial connection.
A TTL-signal port "MS" is connected to the MS instrument used for ligand analysis.

### Transfer component of the interface

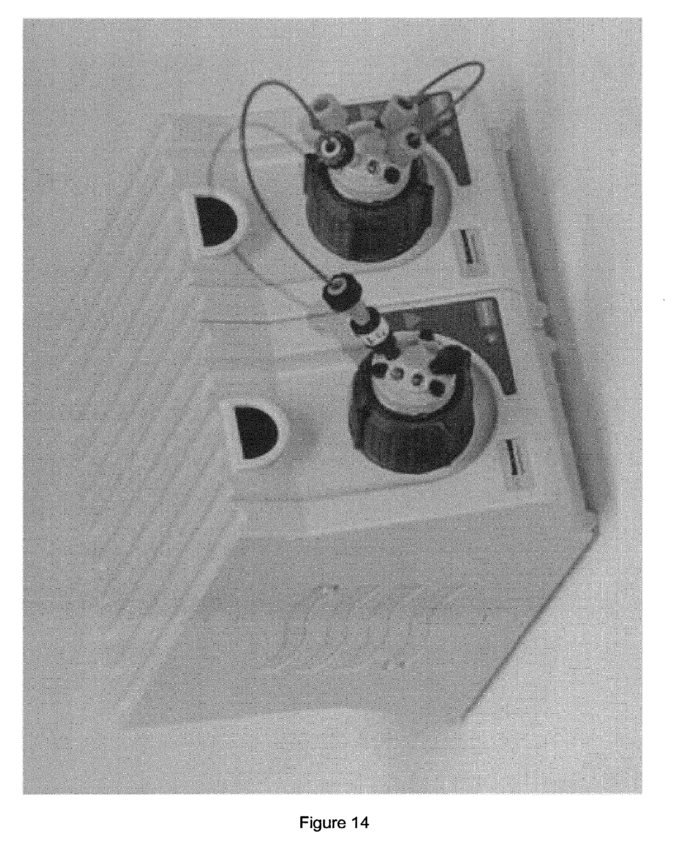

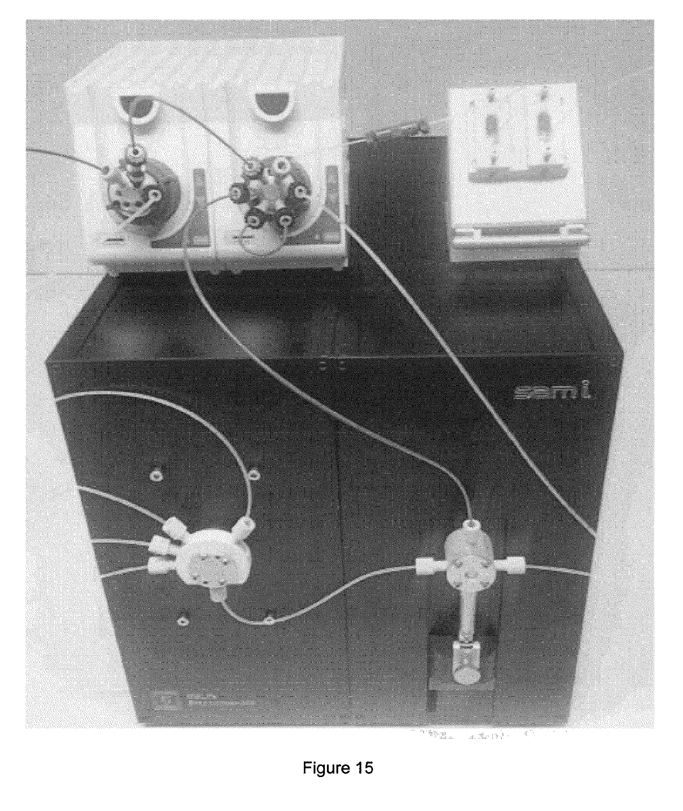

The MS transfer component (Figure 14) consists of two automated, USB controlled MX Rheodyne Valves and a guard microcolumn for buffer exchange, sample desalting and sample concentration. The fluidics connection details and the positions of the valves are described in Example 4. The material of the guard column is typically matched to the sample type (e.g., C4 or C18 column).

### Software of the interface and system connectivity

The hardware components of the affinity-MS interface are controlled during experiments and during maintenance by the SAW2MS software. The exit (usually leading to waste) capillary of the biosensor is connected to the entry of the affinity-chip of the SAW-MS interface. Alternatively, the exit capillary can be directly connected to the MS-transfer component. The capillary directing the sample to the MS is fixed to the inlet of the ESI ion source of the mass spectrometer using appropriate fitting. The further connections are as described in Figure 9.

The USB control cables of the Rheodyne valves are connected to the solvent delivery unit, with their power connectors plugged in to the power adapters. The serial communication cable from the biosensor is connected to the solvent delivery unit according to Figure 14. The biosensor controlling computer is connected through a USB cable to the solvent delivery unit. The three power plugs on the back of the solvent delivery unit are connected to the power adapters of the pump, of the smart valve and of the relay (MS trigger), respectively.

### Example 4:

### System operation of the microfluidic interface of the present invention

Each bioaffinity sensor system - mass spectrometry system coupling experiment has six stages, with different flow paths through the transfer component, as described in the following.

### Stage 0 - Ready

The system is ready for affinity- elution: While sample from the biosensor not required for MS analysis is directed to waste, the equilibrated column waits for elution. The interface software monitors the biosensor sequence, searching for the elution tag (Figure 3 a).

### Stage 1 - Load

After the elution tag is detected by the MS-interface software, the eluted sample from the affinity chip is directed through the guard column, where it is trapped. The duration of loading is defined by the flow-rate of the biosensor and the dead volume of the tubing between biosensor and interface (Figure 3 b).

### Stage 2 - Desalting

The guard column is washed with water or other suitable solvent in order to remove salts, which are directed to the waste. The flow-rate and duration of rinsing is determined by the user and should consider the chemical stability of the analyte (Figure 3 c).

### Stage 3 - Inject

The sample is eluted from the guard column (typically with 70% acetonitrile) and directed into the MS. The flow-rate can be adapted to the requirements of the MS experiment. The duration of injection can be determined by the user and should incorporate the dead volume of tubing connecting the interface with the MS. As an advanced operation mode, a two-step injection can be performed: Initially (Stage 3a), a high flow-rate is used to transfer the sample from the interface to the inlet of the MS. Afterwards, for the actual injection of the sample into the MS, a slow flow-rate (Stage 3b) is used. A flow-rate of 0 µl/min is selected in step 3a to deactivate this option (Figure 3 d).

### Stage 4 - Clean

The column is washed with 90% aqueous acetonitrile, to remove possible remaining contaminations from the column. The user can adjust the flow-rate and duration of the cleaning step (Figure 3 e).

### Stage 5 - Equilibrate

The column is equilibrated with water to perform a new experiment (Figure 3 f). The user can adjust the flow-rate and duration of the equilibration step.

### Example 5:

### Operation example of the interface of the present invention

As an example of a typical affinity-MS coupling experiment with the interface of the present invention, the affinity system of an antibody - antigen is described in the following. The first step of the coupling experiment is the immobilization of one of the interaction partners (ligand) on the gold chip surface of the biosensor, as well as on the affinity-enrichment chip of the biosensor-MS interface. The chips are treated identically in the following. The immobilization of the ligand-binding component of interest is performed using a suitable linker. Typically, suitable linkers are 16-mercaptohexadecanoinc acid or 11-mercaptoundecanoic acid which form a self assembled monolayer (SAM) on the gold surface. For obtaining a SAM, the chips are immersed overnight in a chloroform solution at a 10 mM concentration of the linker. After washing with chloroform and ethanol and drying, the chips are inserted in the biosensor and in the affinity-enrichment interface. The free carboxyl groups of the linker are activated by a mixture of N-hydroxysuccinimide and EDC. The active ester component reacts with free amino groups from the antibody, forming amide bonds. Unreacted active ester groups are capped with ethanolamine. After the antibody is immobilized, the affinity experiment with the antigen sample is performed. After detection of affinity binding by the biosensor, the ligand is eluted at acidic conditions for transfer to the SAW-MS interface. The elution is performed with one or two injections of 100 µL 0.1 M HCl. The elution time can vary according to the flow rate of the experiment. Extra time is considered automatically for the delay caused by tubing connection volume. The desalting step can be performed with different volumes of solvent, considering the stability of the trapped sample on the guard column. After desalting, the elution of the sample from the column into the ESI ion source is performed with a concentration of typically 70 to 80% acetonitrile. An electrical trigger is sent to the ESI-MS to start and stop the acquisition of mass spectra. Subsequently the column is washed and equilibrated for a new experiment. All parameters (timing & flow rates) of these steps are set by the SAW2MS software.

### Example 6:

### Detailed description of the software

### Main window

The hardware components of the interface are controlled during experiments and during maintenance by the SAW2MS software. It comprises the settings for each step of a coupling experiment ("Coupling Procedure"), the log of all the steps and actions ("Application log"), and two buttons for accessing the settings ("Settings") and for stopping the application ("Exit").

All parameters regarding the coupling experiment are set in the "Coupling Procedure". Under Step 1, SAW chip elution / Column binding, the SAW sequence used by the SAW biosensor are loaded using the "Load SAW sequence" button. From the sequence the elution injection to be monitored is selected. If the chip component of the interface is being used, the "Use dual chip system" is selected. This will account for the higher dead volume between biosensor and column caused by the affinity-enrichment chip system.

Under Step 2, Column desalting, the flow rate and the time of desalting can be set. Under Step 3a, Column elution / MS dead volume the flow rate can be set, while on under Step 3b, Column elution / MS measurement, both flow rate and duration can be set, as well as the type of MS instrument. Under Step 4, Column washing, the parameters for cleaning the column can be set, while under Step 5, Column equilibration, the same can be done for equilibrating the column for a new experiment.

After setting these parameters, by clicking the button "Estimate run time", the time for the whole coupling procedure will be calculated. With buttons "Start" and "Stop" the procedure can be started or stopped. The term "Start" does not refer to the start of elution, but the software is now waiting for the elution from SAW biosensor to start the actual elution step as defined above.

On the right side of the main window all commands and events related to the interface are recorded in the window "Application log". They can be deleted by clicking the "Clear log" or can be saved by clicking on "Export log". Under the "Manual pump control" one can set the solvent, the path of the flow through the interface ("Destination"), as well as the flow rate and the duration. The buttons "Start" and "Stop" will set the pump running or will stop it, while the button "Flush" will wash with solvent the piston of the pump and the "Empty" button will drive the piston to the upper position. The "Exit" button allows closing the application.

### Settings

The button "Settings" will bring up a new window, where different special settings can be set under 4 tabs:
"Preferences" - used for automatically loading last parameters and for requiring confirmation before exiting the application
"Tubing volumes" - used for adjusting the volume of tubing in case of replacement of the originals or other system changes involving tubing modifications. For determining the new values of the tubing, one can use the "Tubing calculator". Note that the real volumes might be different from real values.
"MS acquisition trigger settings" - this is used only during troubleshooting or special maintenance procedures.
"Advanced" - this is used for maintenance. The "Prime system" button will exchange completely the liquids within the solvent delivery system, but will not affect the MS-Transfer components. The "Syringe replacement" will drive the piston in a convenient position for replacement.
The "Apply settings" button will save all modifications, while the "Close" button will close the settings window. The settings can be saved as .xml file by clicking "Export current configuration". The button "Import saved configuration" will load previously saved settings, while "Load default data" will load the default values of the settings.

## Claims

1. A microfluidic interface for the coupling of a bioaffinity sensor system to a mass spectrometry system, said interface comprising:
(a) an affinity enrichment component (2), comprising:
(i) one or more microchips (2a, 2b) for affinity enrichment; and/or
(ii) microbeads for affinity enrichment;
(b) a transfer component, comprising;
(i) a first 6-port low pressure switching valve (3a);
(ii) a microcolumn for the desalting of samples (4); and
(iii) a second 6-port low pressure switching valve (3b);
and
(c) a solvent delivery component, comprising:
(i) a pump system (6); and
(ii) a solvent selection valve (5).

2. The microfluidic interface according to claim 1, wherein
(1) the bioaffinitiy sensor system can be brought into fluid communication with the affinity enrichment component (2) via a capillary (1);
(2) the transfer component can be brought into fluid communication with the mass spectrometry system via a capillary (8);
(3) the affinity enrichment component (2) is fluidly connected to the first 6-port low pressure switching valve (3a);
(4) the affinity enrichment component (2) can be brought into fluid communication with a waste reservoir or with the microcolumn for the desalting of samples (4) via the first 6-port low pressure switching valve (3a);
(5) the microcolumn for the desalting of samples (4) is fluidly connected to the second 6-port low pressure switching valve (3b);
(6) the microcolumn for the desalting of samples (4) can be brought into fluid communication with a waste reservoir or with the mass spectrometry system via the second 6-port low pressure switching valve (3b);
(7) the solvent delivery component is fluidly connected to the first 6-port low pressure switching valve (3a);
(8) the solvent delivery system provides selection between different solvents (7) via the solvent selection valve (5); and
(9) the solvent delivery system can be brought into fluid communication with a waste reservoir or with the microcolumn for the desalting of samples (4) via the first 6-port low pressure switching valve (3a).

3. The microfluidic interface according to claim 1, wherein the one or more microchips for affinity enrichment are selected from the group consisting of gold-coated affinity chips and affinity chips having a chemically modified surface.

4. The microfluidic interface according to claim 3, wherein the surface of the gold-coated affinity chips is coated with a self-assembled monolayer (SAM) of 16-mercaptohexadecanoic acid or 11-mercaptoundecanoic acid for the coupling of polypeptides via covalent attachment of amino functions to said 16-mercaptohexadecanoic acid or 11-mercaptoundecanoic acid.

5. The microfluidic interface according to claim 3, wherein the chemical modification is attachment of long-chain alkyl-thiol groups for the coupling of polypeptides via covalent attachment of amino functions to said alkyl-thiol groups.

6. The microfluidic interface according to any one of claims 1 to 5, wherein the affinity enrichment component (2), comprises two microchips (2a, 2b) for affinity enrichment.

7. The microfluidic interface according to any one of claims 1 to 6, wherein the pump system comprises a precision micro pump.

8. The microfluidic interface according to any one of claims 1 to 7, wherein the pump system is capable of providing flow rates in the range of 360 nl/min to 300 µl/min.

9. The microfluidic interface according to any one of claims 1 to 8, further comprising a control electronics system for
(1) controlling the operation of the first and second 6-port low pressure switching valves (3a, 3b);
(2) controlling the operation of the pump system (6) and the solvent selection valve (5);
(3) providing two-way communication with the bioaffinity sensor system;
(4) providing two-way communication with a computer; and
(5) providing two-way communication with the mass spectrometry system.

10. The microfluidic interface according to claim 9, wherein the computer is running computer-readable instructions for controlling the functions of the interface.

11. The microfluidic interface according to claim 10, wherein the functions of the interface that are controlled by the computer include monitoring sample injection in the bioaffinity sensor system, detection of elution profile and sample flow, triggering of mass spectrometric data acquisition, monitoring elution injection, and switching valves for sample transfer.

12. The microfluidic interface according to any one of claims 1 to 11, wherein the microcolumn for the desalting of samples (4) is a C4 column or a C18 column.

13. The microfluidic interface according to any one of claims 1 to 12, wherein the bioaffinity sensor system is a biosensor, selected from the group, consisting of surface plasmon resonance (SPR) biosensors, quartz crystal microbalance (QCM) biosensors, bio-layer interferometry (BLI) biosensors, and surface acoustic wave (SAW) biosensors.

14. The microfluidic interface according to any one of claims 1 to 13, wherein the mass spectrometry system is selected from the group, consisting of electrospray ionization (ESI) mass spectrometry systems, matrix-assisted laser desorption-ionization (MALDI) mass spectrometry systems, desorption-electrospray (DESI) mass spectrometry systems, Fourier transform ion cyclotron resonance (FTICR) mass spectrometry systems, quadrupol time-of-flight (QTOF) mass spectrometry systems, linear ion trap mass spectrometry systems, and orbitrap mass spectrometry systems.

15. A method for the coupling of a bioaffinity sensor system to a mass spectrometry system, comprising the step of appropriately connecting the bioaffinity sensor system and the mass spectrometry system to the microfluidic interface according to any one of claims 1 to 14.
